# FASCICULE DE BREVET EUROPEEN

(11) **EP 1 339 384 B1**
(45) Date de publication et mention de la délivrance du brevet: **06.09.2006**
(21) Numéro de dépôt: 01998321.2
(22) Date de dépôt: 16.11.2001
(51) Int. Cl.: A61K 8/898, A61K 8/891, A61K 8/29, A61Q 17/04, A61Q 1/02

(54) **COMPOSITION COSMETIQUE CONTENANT DES FILTRES MINERAUX**
KOSMETISCHE ZUSAMMENSETZUNG MIT MINERALFILTERN
COSMETIC COMPOSITION CONTAINING MINERAL FILTERS

(30) Priorité: 30.11.2000 FR 0015517
(43) Date de publication de la demande: 03.09.2003
(73) Titulaire: L'ORÉAL, 75008 Paris (FR)
(72) Inventeur: LORANT, Raluca, F-94320 Thiais (FR); LENNON, Paula, F-69003 Lyon (FR)
(74) Mandataire: Miszputen, Laurent
(86) Numéro de dépôt international: PCT/FR2001/003603
(87) Numéro de publication internationale: WO 2002/043690

(56) Documents cités:
- EP-A- 0 748 624
- EP-A- 0 882 673
- WO-A-97/25971
- US-A- 5 744 126
- US-A- 6 066 326

## Description

La présente invention se rapporte à une composition comprenant un oxyde minéral enrobé et un polydiméthylsiloxane à groupements glycosides, notamment sous la forme d'une émulsion eau-dans-huile, et à l'utilisation de ladite composition, notamment dans le domaine cosmétique, en particulier pour le soin et/ou la protection solaire de la peau, des lèvres et des cheveux.

Dans le domaine cosmétique, il est courant d'utiliser des filtres chimiques pour obtenir des produits de protection solaire. Ces filtres chimiques peuvent être introduits assez facilement dans les émulsions par dispersion dans la phase huileuse ou aqueuse de l'émulsion, selon leur caractère lipophile ou hydrophile.

Pour obtenir des forts indices de protection, il est nécessaire d'augmenter le taux de filtres chimiques. Toutefois, pour des raisons de tolérance, on cherche à éviter d'utiliser un taux trop important de filtres chimiques et on préfère introduire à côté ou à la place des filtres chimiques, des filtres physiques minéraux, en particulier des oxydes métalliques comme par exemple le dioxyde de titane et l'oxyde de zinc qui confèrent d'excellentes propriétés protectrices contre les U.V. et une très bonne tolérance cutanée.

Toutefois, l'introduction de ces oxydes métalliques pose des problèmes d'agrément cosmétique. En effet, les produits de protection solaire les contenant se présentent souvent sous forme d'émulsions plus ou moins épaisses, difficiles à appliquer et à étaler, lourdes et poisseuses. En outre, avec certains filtres minéraux comme le dioxyde de titane, à ces défauts s'ajoute un effet de blanchissement lors de l'étalement sur la peau.

Le document US-A-6 066 326 décrit aussi des compositions cosmétiques ou dermatologiques comportant un agent humectant choisi parmi les polydimethylsiloxanes contenant des groupes glucosides, comme protection solaire.

Par ailleurs, on cherche à obtenir des émulsions de protection solaire qui présentent une texture fluide car la texture fluide les rend plus pratiques, plus faciles à appliquer et plus agréables à utiliser. Toutefois, les émulsions fluides sont aussi plus difficiles à réaliser avec des filtres minéraux car les oxydes métalliques présentent l'inconvénient de provoquer la déstabilisation des émulsions dans lesquelles on veut les introduire, et notamment quand il s'agit d'émulsions très fluides. Et cette difficulté d'introduction d'oxydes métalliques est encore plus grande lorsque le taux d'oxydes dépasse 1% de la composition finale.

Les phénomènes d'instabilité se traduisent en particulier par l'agglomération des particules solides, le crémage et la sédimentation des émulsions; un aspect hétérogène des émulsions, et une évolution de la texture dans le temps, cette évolution se traduisant par un épaississement de la texture qui devient, en outre, granuleuse et hétérogène.

Le but de la présente invention est de surmonter ces inconvénients et de proposer une composition permettant d'obtenir des émulsions stables même si elles sont très fluides, présentant de forts indices de protection grâce à la présence, de filtres minéraux et ayant également un grand agrément cosmétique.

La demanderesse a trouvé de manière surprenante que l'utilisation d'un tensioactif siliconé particulier et d'oxyde métallique comportant un enrobage hydrophobe permettait d'atteindre ce but et notamment d'obtenir une émulsion eau-dans-huile contenant des oxydes métalliques, ayant de bonnes propriétés cosmétiques (toucher léger, frais et riche à la fois) et une bonne stabilité dans le temps, même si l'émulsion est très fluide et même si elle contient une proportion importante d'oxydes métalliques. On n'obtient pas des émulsions fluides stables avec des tensioactifs siliconés classiques tels que les alkyldiméthicone copolyols du type Abil EM90.

Aussi, la présente invention a pour objet une composition **sous forme d'émulsion** contenant dans un milieu physiologiquement acceptable, **de 7 à 15% en poids de matière active par rapport au poids total de la composition d'**au moins un oxyde métallique à enrobage hydrophobe et **de 0,1 à 20 % en poids de matière active par rapport au poids total de la composition d'**au moins un polydiméthylsiloxane oxyalkyléné ayant au moins un reste glycoside.

On entend dans la présente demande par « milieu physiologiquement acceptable » un milieu compatible avec la peau y compris le cuir chevelu, les muqueuses et/ou les yeux.

Par ailleurs, on entend ici par « enrobage hydrophobe » un enrobage n'ayant pas d'affinité avec l'eau et ne se laissant pas mouiller par l'eau. Cet enrobage est obtenu par un ou plusieurs traitements de surface de l'oxyde métallique par un ou plusieurs composés hydrophobes.

L'invention a plus particulièrement pour objet une émulsion eau-dans-huile comprenant dans un milieu physiologiquement acceptable, au moins un oxyde métallique à enrobage hydrophobe, et au moins un polydiméthylsiloxane oxyalkyléné ayant au moins un reste glycoside.

Les compositions obtenues selon l'invention, et notamment lorsqu'elles sont sous forme d'émulsions, présentent l'avantage de s'étaler facilement et d'être absorbées rapidement et complètement dans la peau. En même temps qu'un toucher riche et nutritif, elles apportent une sensation de fraîcheur surprenante. Après pénétration du produit, la peau reste douce et mate.

Par ailleurs, les compositions selon l'invention peuvent contenir un pourcentage important de filtres physiques et donc donner un SPF (facteur de protection solaire) élevé tout en étant parfaitement stables et agréables à utiliser.

Le polydiméthylsiloxane oxyalkyléné ayant au moins un reste glycoside peut être par exemple choisi parmi les composés de formule (I) :

R¹ₓR₃₋ₓ-SiO-[SiRR¹O)ₘ-(SiR₂O)ₙ]_{y}-SiR₃₋ₓR¹ₓ (I)

dans laquelle :
m peut être identique ou différent et est 0 ou un nombre de 1 à 200,
n peut être identique ou différent et est 0 ou un nombre de 1 à 1000,
x est 0 ou 1,
y est 0 ou un nombre de 1 à 1200,
R peut être identique ou différent et représente un atome d'hydrogène ou un radical hydrocarboné, éventuellement substitué, comportant de 1 à 18 atomes de carbone,
R¹ peut être identique ou différent et représente un radical de formule (II) :

   Z-(R²O-)_{c}-R³ (II)

   dans laquelle :
   Z représente un reste glycosidique dérivant de 1 à 10 unités monosaccharides ou oligosaccharides,
   R² peut être identique ou différent et représente un radical alcoylène,
   c est 0 ou un nombre de 1 à 20,
   R³ représente un radical alcoylène,
   à la condition que le composé de formule (I) comporte au moins un radical R¹.

Dans la formule (II), Z est de préférence un reste dérivé de monosaccharides. Comme monosaccharides dont peut dériver Z, on peut citer notamment les hexoses et les pentoses, tels que par exemple glucose, fructose, galactose, mannose, talose, allose, altrose, arabinose, xylose, lyxose et ribose. De manière préférée, Z est un reste dérivé du glucose.

Les composés de formule (I) utilisés dans la composition de l'invention peuvent être préparés par tout procédé approprié et notamment selon le procédé décrit dans le document EP-A-612769 incorporé ici pour référence, par préparation d'un glycoside obtenu par réaction d'un mono-saccharide ou d'un oligosaccharide avec un composé de formule (III) :

HO-(R²O-)_{c}-R⁴ (III)

dans laquelle R⁴ représente un radical alcoylène,
et réaction du glycoside obtenu avec un composé organosilicié approprié.

Le polydiméthylsiloxane oxyalkyléné ayant au moins un reste glycoside peut être utilisé tel quel ou en mélange avec un milieu huileux, et notamment dans une ou plusieurs huiles de silicone, plus particulièrement une ou plusieurs huiles de silicone volatiles.

Comme composé de formule (I), on peut utiliser notamment un composé dans lequel un des R représente un groupe octyle et Z-(R²O-)_{c}-R³ représente un groupe dérivé du glucose (Z= reste glucosidique), avec R²O est CH₂O et R³ est un groupe propyle (CH₂)₃. Il peut s'agir plus particulièrement du polydiméthylsiloxane à groupements propyl-polyéthoxyle diglucoside et octyle, en dispersion à 20% de matière active dans du cyclopentadimethylsiloxane (nom CTFA : cyclopentasiloxane / caprylyl-dimethicone ethoxyglucoside) commercialisé sous la dénomination SPG 128 par la société Wacker.

La composition selon l'invention peut contenir une quantité de polydiméthylsiloxane oxyalkyléné ayant un reste glycoside, allant de préférence de 0,5 à 10 % en poids de matière active par rapport au poids total de la composition.

Les oxydes métalliques utilisables dans le cadre de la présente invention sont tous ceux déjà connus en soi pour leur activité photoprotectrice. Ainsi, ils peuvent être notamment choisis parmi les oxydes de titane (dioxyde de titane amorphe ou cristallisé sous forme rutile et/ou anatase), de zinc, de fer, de zirconium, de cérium ou leurs mélanges.

Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence dans la composition de l'invention des nano-pigments.

Les oxydes métalliques à enrobage hydrophobe, utilisés selon l'invention peuvent par exemple avoir subi un ou plusieurs traitements avec un ou plusieurs composés choisis parmi l'alumine, la silice, les dérivés de l'aluminium (par exemple stéarate et laurate), les composés du silicium (par exemple silicones, polydiméthylsiloxanes, alcoxysilanes, siloxy-silicates), les composés du sodium, les oxydes de fer, les esters de fer (par exemple stéarate), les acides gras, les alcools gras et leurs dérivés (tels que l'acide stéarique, l'acide hydroxystéarique, l'alcool stéarylique, l'alcool hydroxystéarylique, l'acide laurique et leurs dérivés), la lécithine, les cires (par exemple la cire de carnauba), les polymères (méth)acryliques (par exemple les polyméthylmethacrylates), les composés fluorés (par exemple les composés perfluoroalkyle et les perfluoroalkyle éthers). Les oxydes peuvent aussi être traités par un mélange de ces composés ou ils peuvent comprendre plusieurs de ces enrobages successifs.

De manière préférée, les oxydes métalliques enrobés utilisés dans la composition de l'invention sont choisis parmi les oxydes de titane, les oxydes de zinc et leurs mélanges.

Plus particulièrement, les oxydes métalliques à enrobage hydrophobe utilisés dans la composition de l'invention peuvent être choisis parmi les oxydes et nano-oxydes de titane traités par :
- l'alumine et l'acide stéarique, comme le produit commercialisé sous la dénomination UV-Titan M160 par la société Kemira, et le produit commercialisé sous la dénomination ST-430C par la société Inanata ;
- les polydiméthylsiloxanes (PDMS), comme les produits commercialisés sous la dénomination Eusolex T-2000 par la société Merck, sous la dénomination UV Titan X170 par la société Eckart, sous la dénomination Si-UFTR-Z par la société Myoshi ;
- les alcoxysilanes, comme le produit commercialisé sous la dénomination Covascreen T1 par la société Wackherr ;
- les composés perfluoroalkyle, comme le produit commercialisé sous la dénomination PF-7 TiO2 MT-600B par la société Daito,
- les perfluoroalkyl éthers, comme le produit commercialisé sous la dénomination TiO2 VF-25-3A par la société Toshiki ;
- les siloxy-silicates, par exemple comme le produit commercialisé sous la dénomination TSS-1 par la société ISK;
- les polyméthylméthacrylates, comme par exemple le produit commercialisé sous la dénomination PW COVASIL S par la société Wacker ;
- la lécithine, comme le produit commercialisé sous la dénomination DUOTERC CW 5-25 par la société Sachtleben ;
- la cire de carnauba, comme le produit commercialisé sous la dénomination UVT-PT 951101 par la société Kemira ;
- la silice et l'alumine, comme les produits commercialisés sous les dénominations Microtitanium Dioxide MT 500 SA et Microtitanium Dioxide MT 100 SA par la société TAYCA, et Tioveil Fin, Tioveil.OP, Tioveil MOTG et Tioveil IPM par la société Uniqema ;
- l'alumine et le stéarate d'aluminium, comme le produit commercialisé sous la dénomination Microtitanium Dioxide MT 100 T par la société Tayca ;
- l'alumine et le laurate d'aluminium, comme le produit commercialisé sous la dénomination Microtitanium Dioxide MT 100 S par la société Tayca ;
- des oxydes de fer et le stéarate de fer, comme le produit commercialisé sous la dénomination Microtitanium Dioxide MT 100 F par la société Tayca ;
- la silice, l'alumine et la silicone, comme les produits commercialisés sous les dénominations Microtitanium Dioxide MT 100 SAS, Microtitanium Dioxide MT 600 SAS et Microtitanium Dioxide MT 500 SAS par la société Tayca ;
- l'octyltriméthoxysilane, comme le produit commercialisé sous la dénomination T-805 par la société Degussa ;
- l'alumine et la glycérine, comme le produit commercialisé sous la dénomination UVT-M212 par la société Kemira;
- l'alumine et la silicone, comme le produit commercialisé sous la dénomination UVT-M262 par de la société Kemira.

Ces oxydes et nano-oxydes de titane enrobés hydrophobes peuvent se présenter sous forme de charge solide ou sous forme de dispersion dans un milieu huileux. Comme dispersions d'oxyde de titane enrobé, on peut citer par exemple les produits indiqués ci-dessus, commercialisés par la société Uniqema sous les dénominations TIOVEIL FIN (nano-oxyde de titane dispersé dans benzoate d'alcool C12-C15 avec, comme dispersant, un polycondensat d'acide hydrostéarique) et TIOVEIL MOTG (nano-oxyde de titane dispersé dans huile minérale/triglycérides avec, comme dispersant, un polycondensat d'acide hydroxystéarique) ; le produit commercialisé sous la dénomination COVASCREEN TI par la société Wacker (dispersion huileuse de TiO2 enrobé par du triméthoxy-octylsilane à 60 %) ; le produit commercialisé sous la dénomination DAITOPERSION TI-30 par la société Daito (dispersion de nano-oxyde de titane enrobé par du polyméthylhydrogènesiloxane dans cyclométhicone et diméthicone copolyol) ; le produit commercialisé sous la dénomination TIOSPERSE ULTRA par la société Collaborative Laboratories (nano-oxyde de titane enrobé par acide stéarique/alumine, dispersé dans du benzoate d'hydroxystéarate d'éthyl-2-hexyle) ; le produit commercialisé sous la dénomination MIBRID DISPERSION SAS4 5050 par la société Myoshi (nano-oxyde de titane enrobé par alumine/acide stéarique puis par polydiméthylsiloxane, dispersé dans cyclopentasiloxane) ; le produit commercialisé sous la, dénomination SPD-T1 par la société Shin-Etsu (nano-oxyde de titane enrobé par un polymère acrylique greffé silicone et dispersé dans le cyclopentadiméthylsiloxane).

Selon un mode préféré de réalisation de l'invention, on utilise dans la composition de l'invention les oxydes de titane suivants : SI-UFTR-Z, TIOVEIL MOTG, UV-TITAN M160, EUSOLEX T-2000, PF-7 TIO2 MT-600B, PW COVASIL S, TSS-1, COVASCREEN TI, DAITOPERSION TI-30, TIOSPERSE ULTRA, UV-TITAN X 170, MIBRID DISPERSION SAS4 5050, SPD-T1, TIOVEIL FIN, et plus préférentiellement les produits UV-TITAN X 170, TIOSPERSE ULTRA, PW COVASIL S, MIBRID DISPERSION SAS4 5050, SPD-T1.

Comme oxyde de zinc utilisables dans la composition de l'invention, on peut citer par exemple les dispersions de nano-oxydes de zinc commercialisées sous les dénominations DAITOPERSION ZN-30 et Zn-50 par la société Daito, qui sont des dispersions dans du cyclopolyméthylsiloxane / polydiméthylsiloxane oxyéthyléné, contenant 30% ou 50% de nano-oxydes de zinc enrobés par le polyméthylhydrogènesiloxane.

On peut utiliser une ou plusieurs sortes d'oxydes métalliques enrobés dans la composition de l'invention.

La composition de l'invention est destinée à une application topique et plus particulièrement à une application sur la peau, les cheveux et/ou les muqueuses. Elle peut constituer notamment une composition cosmétique et/ou dermatologique.

Cette composition peut se présenter sous forme d'une émulsion obtenue par dispersion d'une phase grasse (huileuse) dans une phase aqueuse (H/E) ou inversement (E/H), ou d'une émulsion multiple et par exemple d'une émulsion triple (E/H/E ou H/E/H), ou sous forme d'une dispersion vésiculaire de type ionique (liposomes) et/ou non ionique. Ces compositions sont préparées selon les méthodes usuelles.

La composition de l'invention peut constituer par exemple une lotion, un lait ou une crème plus ou moins fluide. Elle peut même être très fluide sans être déstabilisée. On entend ici par « très fluide » une composition ayant une viscosité allant d'environ 60 à 600 cPoises (60 à 600 mPa.s).

La composition selon l'invention comprend généralement une phase huileuse, qui peut être constituée par tous les corps gras et notamment les huiles, classiquement utilisés dans le domaine cosmétique. Selon un mode préféré de réalisation de l'invention, la composition comprend au moins une huile.

Parmi les huiles utilisables dans la composition de l'invention, on peut citer par exemple les huiles végétales telles que l'huile de noyaux d'abricot et l'huile de soja ; les huiles minérales comme l'huile de vaseline ; les huiles de synthèse comme l'isohexadécane ; les huiles de silicone volatiles ou non volatiles et les huiles fluorées. Comme huiles de silicone volatiles, on peut citer notamment les polydiméthylsiloxanes cycliques ou cyclométhicones qui comportent d'environ 3 à 9 atomes de silicium et de préférence de 4 à 6 atomes de silicium, telles que le cyclohexadiméthylsiloxane (ou cyclohexaméthicone) et le cyclopentadiméthylsiloxane (ou cyclopentaméthicone), et les polydiméthylsiloxanes linéaires volatiles comportant d'environ 3 à 9 atomes de silicium. Selon un mode préféré de réalisation de l'invention, la composition comprend au moins une huile de silicone.

Les autres corps gras susceptibles d'être présents dans la phase huileuse peuvent être par exemple les acides gras, les alcools gras et les cires telles que la vaseline ou la cire d'abeille.

Quand la composition est anhydre, la quantité de phase huileuse représente le complément à 100 % en poids, des quantités de polydiméthylsiloxane oxyalkyléné ayant au moins un groupement glycoside et d'oxyde métallique, c'est-à-dire que la quantité de phase huileuse peut aller par exemple de 50 à 99,4 % en poids par rapport au poids total de la composition. La composition se présente

sous forme d'une émulsion et plus spécialement sous forme d'une émulsion E/H, notamment sous forme d'une émulsion fluide, c'est-à-dire présentant une viscosité allant de 60 à 600 cP (60 à 600 mPa.s) et encore mieux de 80 et 250 cP (80 à 250 mPa.s), la viscosité étant mesurée au viscosimètre Metler RM 180 (Rheomat) avec un mobile M2, à 25°C et à une vitesse de 200 tours /min. On obtient ainsi une émulsion assez fluide, très agréable à utiliser car elle s'étale facilement et de façon homogène sans laisser de sensation de gras ou de film rêche ou collant.

La quantité de phase huileuse dans les émulsions et en particulier dans une émulsion E/H selon l'invention, peut aller par exemple de 5 à 80 % et de préférence de 40 à 70 % en poids par rapport au poids total de l'émulsion. La phase aqueuse de l'émulsion peut représenter par exemple de 50 à 95 % et de préférence de 60 à 90 % en poids par rapport au poids total de l'émulsion.

De façon connue, la composition de l'invention peut contenir également des adjuvants habituels dans le domaine cosmétique, tels que les actifs, les conservateurs, les antioxydants, les agents complexants, les solvants, les parfums, les charges, les filtres solaires, les bactéricides, les électrolytes (tels que sulfate de magnésium), les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase huileuse, dans la phase aqueuse et/ou dans les vésicules lipidiques. Ces adjuvants ainsi que leurs concentrations doivent être tels qu'ils ne modifient pas la propriété recherchée pour la composition de l'invention.

Selon un mode préféré de réalisation de l'invention, la composition comprend au moins un filtre solaire. Comme filtres solaires, la composition de l'invention peut comprendre tous les filtres UVA et UVB utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX;
(3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
(4) les dérivés de l'acide p-aminobenzoique ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres. UVA, on peut citer par exemple:
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) les filtres actifs dans l'UV-A de formule (IV) suivante : 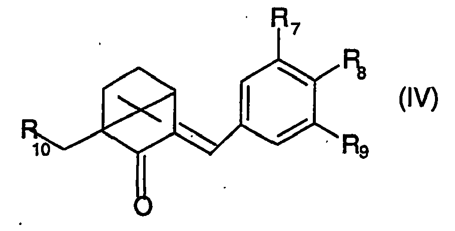 dans laquelle :
   - R₇ et R₉, identiques ou différents, désignent un hydrogène, un halogène, un radical OH, un radical alkyle, saturé ou non, linéaire ou ramifié en C₁-C₁₀ ; un radical alcoxy, saturé ou non, linéaire ou ramifié en C₁-C₁₀ ou un groupe HSO₃ ;
   - R₁₀ désigne un hydrogène ou HSO₃;
   - R₈ désigne un groupe OH ; un groupe OR₁₁ où R₁₁ désigne un radical alkyle, saturé ou insaturé, linéaire ou ramifié en C₁-C₁₀ ; ou bien un groupe de structure suivante : 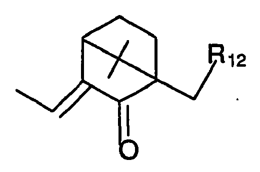
   dans laquelle R₁₂ désigne hydrogène ou HSO₃ ;
   ou bien un groupe de formule suivante : 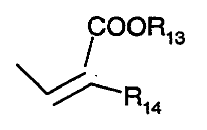 ou bien un groupe de formule suivante : 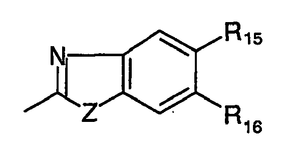 dans lesquelles :
   - Z désigne un atome d'oxygène ou un radical -NH- ;
   - R₁₃, R₁₄, R₁₅ et R₁₆, identiques ou différents, désignent un hydrogène, un halogène, un radical OH, un radical alkyle, saturé ou non, linéaire ou ramifié en C₁-C₁₀; un radical alcoxy, saturé ou non, linéaire ou ramifié en C₁-C₁₀ ou un groupe HSO₃,

   On peut citer en particulier comme composé de formule (IV), l'acide benzène 1,4 [di(3-méthylidènecampho-10-sulfonique)] éventuellement sous forme partiellement ou totalement neutralisée qui répond à la formule (V) suivante : 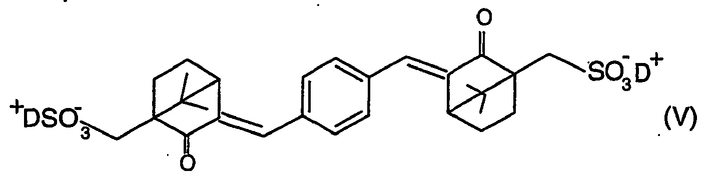 dans laquelle D désigne un atome d'hydrogène, un métal alcalin ou encore un radical NH(R₂₅)₃⁺ dans lequel les radicaux R₂₅, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺, et en particulier l'acide benzène 1,4-di(3-méthylidène-10-campho-sulfonique) commercialisé sous la dénomination MEXORYL SX par la société Chimex.
(3) les dérivés de benzophénone, par exemple :
   - la 2,4-dihydroxybenzophénone (benzophénone-1);
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-métlioxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels de structure suivante (VI) : 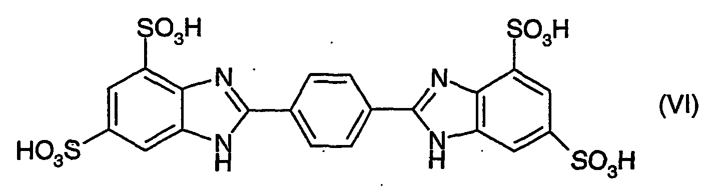 commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthyl-1-[(triméthylsilyl)oxy]disiloxanyl]propyl]-1H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolyl-benzoxazole;
   - le 2-[1-(3-trimethylsilanyl-propyl)-1H-benzimidazol-2-yl]-benzothiazole ; qui sont décrits dans la demande de brevet EP-A-1 028 120 ;
(8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

On peut aussi utiliser un mélange de plusieurs de ces filtres et un mélange de filtres UVB et de filtres UVA.

La composition selon l'invention trouve son application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

La composition selon l'invention peut par exemple être utilisée comme produit de soin et/ou de protection solaire pour le visage et/ou le corps sous forme de crèmes ou de laits.

Aussi, l'invention a encore pour objet l'utilisation cosmétique de la composition telle que définie ci-dessus pour le soin et/ou de protection solaire de la peau, des lèvres et/ou des cheveux, et/ou pour le maquillage de la peau et/ou des lèvres.

L'invention a aussi pour objet un procédé de traitement cosmétique pour protéger la peau, y compris le cuir chevelu, les cheveux, et/ou les lèvres contre le rayonnement solaire, caractérisé en ce qu'il consiste à appliquer sur la peau, les cheveux et/ou les lèvres, une composition telle que définie ci-dessus.

Les exemples ci-après d'émulsions selon l'invention sont donnés à titre d'illustration et sans caractère limitatif. Les quantités y sont données en % en poids, sauf mention contraire.

### Exemple 1 : Emulsion E/H de SPF 40

### Phase huileuse :

- PDMS à groupement propyle polyéthoxylé diglucoside et octyl à 20% dans le cyclopentadimethylsiloxane (SPG 128 de Wacker) 20 %
   (soit 4 % de M.A.)
- Cyclohexadimethylsiloxane 20 %
- p-methoxy cinnamate (Parsol MCX) (filtre liposoluble) 7 %
- Dioxyde de titane enrobé PDMS (UV Titan X170 de la société Kemira) 10 % (en M.A.)

### Phase aqueuse

- Glycérine 5 %
- Conservateurs qs %
- Mexoryl SX (filtre hydrosoluble) 3 %
- Eau déminéralisée qsp 100 %

Mode opératoire : on homogénéise chacune des deux phases puis on les mélange sous agitation en dispersant de la phase aqueuse dans la phase huileuse.

On obtient un lait fluide très doux, ne blanchissant pas à l'application. Son aspect au microscope est fin et régulier et on observe une bonne dispersion des pigments.

Cette émulsion reste stable après deux mois de conservation à 45°C.

### Exemple comparatif 1 : Emulsion E/H avec oxyde de titane non enrobé

On reproduit l'exemple 1, mais en remplaçant le dioxyde de titane enrobé par la même quantité de dioxyde de titane non enrobé (TITANIUM DIOXYDE P25 commercialisé par la société Degussa).

On obtient une émulsion grossière et instable : il se produit une coalescence des gouttes d'eau et un relargage d'eau et d'huile quelques heures après la préparation de l'émulsion. L'étude de l'émulsion au microscope confirme cette instabilité.

### Exemple comparatif 2 : Emulsion E/H avec un alkyldiméthicone copolyol

On reproduit l'exemple 1, mais en remplaçant le PDMS à groupement propyle polyéthoxylé diglucoside et octyl, par la même quantité de matière active (4 %) d'un alkyl diméthicone copolyol (cétyldiméthicone copolyol: Abil EM90 de Goldschmidt).

On obtient une émulsion instable : il se produit, dans le temps, une sédimentation des gouttelettes de phase aqueuse et du dioxyde de titane qui ne sont plus répartis de manière homogène dans la phase huileuse, et de ce fait il se produit aussi une apparition d'huile en surface.

### Exemple 2 : Fond de teint fluide SPF 20

### Phase huileuse

- PDMS à groupement propyle polyéthoxylé diglucoside et octyl à 20% dans le cyclopentadimethylsiloxane (SPG 128 de Wacker) 20 %
   (soit 4 % de M.A.)
- Cyclopentadimethylsiloxane 30 %
- Pigments bruns, rouges, jaunes 5 %
- TiO₂ enrobé (TIOSPERSE ULTRA) 15 % (en M.A.)

### Phase aqueuse

- Conservateur qs
- Mexoryl SX (filtre hydrosoluble) 3 %
- Eau qsp 100 %

Mode opératoire : on homogénéise les deux phases puis on fait l'émulsion sous agitation par dispersion de la phase aqueuse dans la phase huileuse.

On obtient un fond de teint fluide très doux, laissant la peau satinée. Son aspect au microscope est fin, régulier, on observe une bonne dispersion des pigments.

Cette émulsion reste stable après deux mois de conservation à 45°C.

## Revendications

1. Composition **sous forme d'émulsion** contenant dans un milieu physiologiquement acceptable **de 7 à 15% en poids de matière active par rapport au poids total de la composition d'**au moins un oxyde métallique à enrobage hydrophobe et **de 0,1 à 20 % en poids de matière active par rapport au poids total de la composition d'**au moins un polydiméthylsiloxane oxyalkyléné ayant au moins un reste glycoside.

2. Composition selon la revendication 1, **caractérisée en ce que** le polydiméthylsiloxane oxyalkyléné ayant au moins un reste glycoside est choisi parmi les composés de formule (I) :
R¹ₓR₃₋ₓ-SiO-[SiRR¹O)ₘ-(SiR₂O)ₙ]_{y}-SiR₃₋ₓR¹ₓ (I)
dans laquelle :
m peut être identique ou différent et est 0 ou un nombre de 1 à 200,
n peut être identique ou différent et est 0 ou un nombre de 1 à 1000,
x est 0 ou 1,
y est 0 ou un nombre de 1 à 1200,
R peut être identique ou différent et représente un atome d'hydrogène ou un radical hydrocarboné, éventuellement substitué, comportant de 1 à 18 atomes de carbone,
R¹ peut être identique ou différent et représente un radical de formule (II) :
Z-(R²O-)_{c}-R³ (II)
dans laquelle :
Z représente un reste glycosidique dérivant de 1 à 10 unités monosaccharides ou oligosaccharides,
R² peut être identique ou différent et représente un radical alcoylène,
c est 0 ou un nombre de 1 à 20,
R³ représente un radical alcoylène,
à la condition que le composé de formule (I) comporte au moins un radical R¹.

3. Composition selon la revendication précédente, **caractérisée en ce que** Z est un reste dérivé de monosaccharides choisis parmi les hexoses et les pentoses.

4. Composition selon la revendication précédente, **caractérisée en ce que** Z est un reste dérivé de glucose.

5. Composition selon l'une quelconque des revendications 2 à 4, **caractérisée en ce que** dans la formule (I), un des R représente un groupe octyle et Z-(R²O-)_{c-}R³ représente un groupe dérivé du glucose, R²O étant CH₂O et R³ étant un groupe propyle.

6. Composition selon l'une quelconque des revendications précédentes, **caractérisée en ce que** le polydiméthylsiloxane oxyalkyléné ayant au moins un reste glycoside est en mélange avec une ou plusieurs huiles de silicone.

7. Composition selon l'une quelconque des revendications précédentes, **caractérisée en ce que** la quantité de polydiméthylsiloxane oxyalkyléné à groupement glycoside va de 0,5 à 10 % en poids de matière active par rapport au poids total de la composition.

8. Composition selon l'une quelconque des revendications précédentes, **caractérisée en ce que** l'oxyde métallique à enrobage hydrophobe est choisi parmi les oxydes de titane, de zinc, de fer, de zirconium, de cérium ou leurs mélanges.

9. Composition selon l'une quelconque des revendications précédentes, **caractérisée en ce que** l'oxyde métallique à enrobage hydrophobe est sous forme de nano-pigment.

10. Composition selon l'une quelconque des revendications précédentes, **caractérisée en ce que** l'oxyde métallique à enrobage hydrophobe a subi un ou plusieurs traitements avec un ou plusieurs composés choisis parmi l'alumine, la silice, les dérivés de l'aluminium, les composés du silicium, les composés du sodium, les oxydes de fer, les esters de fer, les acides gras, les alcools gras et leurs dérivés, la lécithine, les cires, les polymères (méth)acryliques, les composés fluorés.

11. Composition selon l'une quelconque des revendications précédentes, **caractérisée en ce que** l'oxyde métallique enrobé est choisi parmi les oxydes de titane, les oxydes de zinc et leurs mélanges.

12. Composition selon l'une quelconque des revendications précédentes, **caractérisée en ce qu'**elle contient en outre au moins une huile.

13. Composition selon l'une quelconque des revendications précédentes, **caractérisée en ce qu'**elle contient en outre un filtre solaire.

14. Composition selon l'une quelconque des revendications précédentes, **caractérisée en ce qu'**elle constitue une émulsion eau-dans-huile.

15. Composition selon la revendication 14, **caractérisée en ce que** la phase huileuse représente de 5 à 80 % en poids par rapport au poids total de la composition.

16. Composition selon l'une quelconque des revendications précédentes, **caractérisée en ce qu'**elle constitue un produit de soin ou de protection solaire pour le visage et/ou le corps.

17. Procédé de traitement cosmétique pour protéger la peau, les cheveux, et/ou les lèvres contre le rayonnement solaire, **caractérisé en ce qu'**il consiste à appliquer sur la peau, les cheveux et/ou les lèvres, une composition selon l'une quelconque des revendications 1 à 16.

## Claims

1. Composition in the form of an emulsion containing, in a physiologically acceptable medium, from 7% to 15% by weight of active material relative to the total weight of the composition of at least one metal oxide with a hydrophobic coating and from 0.1% to 20% by weight of active material relative to the total weight of the composition of at least one oxyalkylenated polydimethylsiloxane containing at least one glycoside residue.

2. Composition according to Claim 1, **characterized in that** the oxyalkylenated polydimethylsiloxane containing at least one glycoside residue is chosen from the compounds of formula (I):
R¹ₓR₃₋ₓ-SiO-[SiRR¹O)ₘ-(SiR₂O)ₙ]_{y}-SiR₃₋ₓR¹ₓ (I)
in which:
m may be identical or different and is 0 or a number from 1 to 200,
n may be identical or different and is 0 or a number from 1 to 1 000,
x is 0 or 1,
y is 0 or a number from 1 to 1 200,
R may be identical or different and represents a hydrogen atom or an optionally substituted hydrocarbon-based radical containing from 1 to 18 carbon atoms,
R¹ may be identical or different and represents a radical of formula (II):
Z-(R²O-)_{c}-R³ (II)
in which:
Z represents a glycoside residue derived from 1 to 10 monosaccharide or oligosaccharide units,
R² may be identical or different and represents an alkylene radical,
c is 0 or a number from 1 to 20,
R³ represents an alkylene radical,
with the proviso that the compound of formula (I) comprises at least one radical R¹.

3. Composition according to the preceding claim, **characterized in that** Z is a residue derived from monosaccharides chosen from hexoses and pentoses.

4. Composition according to the preceding claim, **characterized in that** Z is a residue derived from glucose.

5. Composition according to any one of Claims 2 to 4, **characterized in that**, in formula (I), one of the radicals R represents an octyl group and Z-(R²O-)_{c-}R³ represents a group derived from glucose, R²O being CH₂O and R³ being a propyl group.

6. Composition according to any one of the preceding claims, **characterized in that** the oxyalkylenated polydimethylsiloxane containing at least one glycoside residue is in mixture with one or more silicone oils.

7. Composition according to any one of the preceding claims, **characterized in that** the amount of oxyalkylenated polydimethylsiloxane containing a glycoside group ranges from 0.5% to 10% by weight of active material relative to the total weight of the composition.

8. Composition according to any one of the preceding claims, **characterized in that** the metal oxide with a hydrophobic coating is chosen from titanium oxide, zinc oxide, iron oxide, zirconium oxide and cerium oxide, or mixtures thereof.

9. Composition according to any one of the preceding claims, **characterized in that** the metal oxide with a hydrophobic coating is in the form of a nano-pigment.

10. Composition according to any one of the preceding claims, **characterized in that** the metal oxide with a hydrophobic coating has undergone one or more treatments with one or more compounds chosen from alumina, silica, aluminium derivatives, silicon compounds, sodium compounds, iron oxides, iron esters, fatty acids, fatty alcohols and derivatives thereof, lecithin, waxes, (meth)acrylic polymers and fluoro compounds.

11. Composition according to any one of the preceding claims, **characterized in that** the coated metal oxide is chosen from titanium oxides and zinc oxides, and mixtures thereof.

12. Composition according to any one of the preceding claims, **characterized in that** it also contains at least one oil.

13. Composition according to any one of the preceding claims, **characterized in that** it also contains a sunscreen.

14. Composition according to any one of the preceding claims, **characterized in that** it is a water-in-oil emulsion.

15. Composition according to Claim 14, **characterized in that** the oily phase represents from 5% to 80% by weight relative to the total weight of the composition.

16. Composition according to any one of the preceding claims, **characterized in that** it is an antisun care or protection product for the face and/or the body.

17. Cosmetic treatment process for protecting the skin, the hair and/or the lips against solar radiation, **characterized in that** it consists in applying to the skin, the hair and/or the lips a composition according to any one of Claims 1 to 16.

## Patentansprüche

1. In Form einer Emulsion vorliegende Zusammensetzung, die in einem physiologisch akzeptablen Medium 7 bis 15 Gew.-% wirksame Substanz mindestens eines Metalloxids mit hydrophober Umhüllung, bezogen auf das Gesamtgewicht der Zusammensetzung, und 0,1 bis 20 Gew.-% wirksame Substanz mindestens eines alkoxylierten Polydimethylsiloxans mit mindestens einem Glycosidrest, bezogen auf das Gesamtgewicht der Zusammensetzung, enthält.

2. Zusammensetzung nach Anspruch 1, **dadurch gekennzeichnet, dass** das alkoxylierte Polydimethylsiloxan mit mindestens einem Glycosidrest unter den Verbindungen der Formel (I) ausgewählt ist:
R¹ₓR₃₋ₓ-SiO-[SiRR¹O)ₘ-(SiR₂O)ₙ]_{y}-SiR₃₋ₓR¹ₓ (I),
worin bedeuten:
die Indizes m, die gleich oder verschieden sind, 0 oder eine Zahl von 1 bis 200,
die Indizes n, die gleich oder verschieden sein können, 0 oder eine Zahl von 1 bis 1 000,
x 0 oder 1,
y 0 oder eine Zahl von 1 bis 1 200,
die Gruppen R, die gleich oder verschieden sein können, ein Wasserstoffatom oder eine gegebenenfalls substituierte Kohlenwasserstoffgruppe mit 1 bis 18 Kohlenstoffatomen,
die Gruppen R¹, die gleich oder verschieden sein können, eine Gruppe der folgenden Formel (II):
Z-(R²O-)_{c}-R³ (II),
worin bedeuten:
Z einen Glycosidrest, der von 1 bis 10 Monosaccharideinheiten oder Oligosaccharideinheiten abgeleitet ist,
die Gruppen R², die gleich oder verschieden sein können, eine Alkylengruppe,
c 0 oder eine Zahl von 1 bis 20,
R³ eine Alkylengruppe
mit der Maßgabe, dass die Verbindung der Formel (I) mindestens eine Gruppe R¹ aufweist.

3. Zusammensetzung nach dem vorhergehenden Anspruch, **dadurch gekennzeichnet, dass** Z ein Rest ist, der von Monosacchariden abgeleitet ist, die unter den Hexosen und Pentosen ausgewählt sind.

4. Zusammensetzung nach dem vorhergehenden Anspruch, **dadurch gekennzeichnet, dass** Z ein von Glucose abgeleiteter Rest ist.

5. Zusammensetzung nach einem der Ansprüche 2 bis 4, **dadurch gekennzeichnet, dass** in der Formel (I) eine der Gruppen R eine Octylgruppe bedeutet und Z-(R²O-)_{c}-R³ eine von Glucose abgeleitete Gruppe ist, wobei R²O CH₂O und R³ Propyl bedeutet.

6. Zusammensetzung nach einem der vorhergehenden Ansprüche, **dadurch gekennzeichnet, dass** das alkoxylierte Polydimethylsiloxan mit mindestens einem Glycosidrest im Gemisch mit einem oder mehreren Siliconölen vorliegt.

7. Zusammensetzung nach einem der vorhergehenden Ansprüche, **dadurch gekennzeichnet, dass** der Mengenanteil des alkoxylierten Polydimethylsiloxans mit Glycosidgruppe im Bereich von 0,5 bis 10 Gew.-%, bezogen auf das Gesamtgewicht der Zusammensetzung, liegt.

8. Zusammensetzung nach einem der vorhergehenden Ansprüche, **dadurch gekennzeichnet, dass** das Metalloxid mit hydrophober Umhüllung unter den Oxiden von Titan, Zink, Eisen, Zirconium, Cer oder deren Gemischen ausgewählt ist.

9. Zusammensetzung nach einem der vorhergehenden Ansprüche, **dadurch gekennzeichnet, dass** das Metalloxid mit hydrophober Umhüllung in Form eines Nanopigments vorliegt.

10. Zusammensetzung nach einem der vorhergehenden Ansprüche, **dadurch gekennzeichnet, dass** das Metalloxid mit hydrophober Umhüllung einer oder mehreren Behandlungen mit einer oder mehreren Verbindungen unterzogen wurde, die ausgewählt sind unter Aluminiumoxid, Siliciumoxid, Aluminiumderivaten, Siliciumderivaten, Natriumverbindungen, Eisenoxiden, Eisenestern, Fettsäuren, Fettalkoholen und deren Derivaten, Lecithin, Wachsen, (Meth)acrylpolymeren und fluorierten Verbindungen.

11. Zusammensetzung nach einem der vorhergehenden Ansprüche, **dadurch gekennzeichnet, dass** das umhüllte Metalloxid unter den Oxiden von Titan und den Oxiden von Zink und deren Gemischen ausgewählt ist.

12. Zusammensetzung nach einem der vorhergehenden Ansprüche, **dadurch gekennzeichnet, dass** sie ferner mindestens ein Öl enthält.

13. Zusammensetzung nach einem der vorhergehenden Ansprüche, **dadurch gekennzeichnet, dass** sie ferner einen Sonnenschutzfilter enthält.

14. Zusammensetzung nach einem der vorhergehenden Ansprüche, **dadurch gekennzeichnet, dass** sie eine Wasser-in-Ö1-Emulsion ist.

15. Zusammensetzung nach Anspruch 14, **dadurch gekennzeichnet, dass** die Ölphase 5 bis 80 Gew.-%, bezogen auf das Gesamtgewicht der Zusammensetzung, ausmacht.

16. Zusammensetzung nach einem der vorhergehenden Ansprüche, **dadurch gekennzeichnet, dass** sie ein Produkt zur Pflege oder für den Sonnenschutz des Gesichts und/oder des Körpers ist.

17. Verfahren zur kosmetischen Behandlung, um die Haut, die Haare und/ oder die Lippen gegen Sonnenlicht zu schützen, **dadurch gekennzeichnet, dass** es darin besteht, auf die Haut, die Haare und/ oder die Lippen eine Zusammensetzung nach einem der Ansprüche 1 bis 16 aufzutragen.
